# EUROPÄISCHE PATENTSCHRIFT

(11) **EP 3 110 393 B1**
(45) Veröffentlichungstag und Bekanntmachung des Hinweises auf die Patenterteilung: **31.10.2018**
(21) Anmeldenummer: 15705313.3
(22) Anmeldetag: 19.02.2015
(51) Int. Cl.: A61K 8/37, A61Q 5/02, A61Q 13/00, A61Q 19/10, C07C 69/738, C07C 69/716, C11B 9/00, C11D 3/50

(54) **PHOTOLABILE DUFTSPEICHERSTOFFE**
PHOTOLABILE PRO-FRAGRANCES
SUBSTANCES ODORIFÉRANTES PHOTOLABILES

(30) Priorität: 24.02.2014 DE 102014203252
(43) Veröffentlichungstag der Anmeldung: 04.01.2017
(73) Patentinhaber: Henkel AG & Co. KGaA, 40589 Düsseldorf (DE)
(72) Erfinder: KROPF, Christian, 40724 Hilden (DE); GERKE, Thomas, 40627 Düsseldorf (DE); HUCHEL, Ursula, 50733 Köln (DE); GRIESBECK, Axel, 50935 Köln (DE); LANDES GEB. BURCZYK, Agnieszka, 50969 Köln (DE)
(74) Vertreter: Viering, Jentschura & Partner mbB Patent- und Rechtsanwälte
(86) Internationale Anmeldenummer: PCT/EP2015/053502
(87) Internationale Veröffentlichungsnummer: WO 2015/124671

(56) Entgegenhaltungen:
- WO-A1-2006/023864
- WO-A1-2008/069609
- WO-A1-2009/055917
- WO-A1-2009/118219
- US-A- 4 325 962
- US-A1- 2011 257 014
- KRAUS G A ET AL: "Michael addition reactions of alpha-acyloxy nitrile anions", TETRAHEDRON LETTERS, PERGAMON, GB, Bd. 41, Nr. 1, 1. Januar 2000 (2000-01-01) , Seiten 21-24, XP004185407, ISSN: 0040-4039, DOI: 10.1016/S0040-4039(99)02017-1
- DUUS, F.: "The acid catalysed reaction of 1,4-diketones with hydrogen sulfide; A convenient route to substituted thiophens", TETRAHEDRON, Bd. 32, 31. Dezember 1976 (1976-12-31), Seiten 2817-2825, XP002738468,
- LIU, W; LIU, J.; OGAWA, D.; NISHIHARA, Y.; GUO, X.;LI, Z.: "Iron-Catalyzed Oxidation of Tertiary Amines: Synthesis of beta-1,3-Dicarbonyl Aldehydes by Three-Component C-C Couplings", ORGANIC LETTERS, Bd. 13, Nr. 23, 11. April 2011 (2011-04-11), Seiten 6272-6275, XP002738469,
- CHOW, Y.; WANG, S.; CHENG, X.: "The photocycoaddition of dibenzoylmethanatoboron difluoride (DBMBF2) with conjugated enones and en-esters", CANADIAN JOURNAL OF CHEMISTRY, Bd. 71, 31. Dezember 1993 (1993-12-31), Seiten 846-854, XP002738470,
- HOFFMAN, R.; KIM, H.: "A New Chiral Alkylation Methodology For The Synthesis of 2-Alkyl-4-Ketoacids In High Optical Purity Using 2-Triflyloxy Esters", TETRAHEDRON LETTERS, Bd. 34, Nr. 13, 31. Dezember 1993 (1993-12-31), Seiten 2051-2054, XP002738471,

## Beschreibung

Die vorliegende Erfindung liegt auf dem Gebiet der Duftspeicherstoffe, wie sie zum Beispiel auf dem Gebiet der Wasch- oder Reinigungsmittel, kosmetischen Mittel sowie Luftpflegemittel Einsatz finden. Die Erfindung betrifft insbesondere spezielle Ketone, die als photolabile Duftspeicherstoffe fungieren. Ferner betrifft die vorliegende Erfindung Wasch- oder Reinigungsmittel, kosmetische Mittel sowie Luftpflegemittel, welche solche Ketone enthalten. Ferner betrifft sie ein Verfahren zur lang anhaltenden Beduftung von Oberflächen und ebenso ein Verfahren zur lang anhaltenden Raumbeduftung.

Wasch- oder Reinigungsmittel bzw. kosmetische Mittel enthalten zumeist Duftstoffe, die den Mitteln einen angenehmen Geruch verleihen. Die Duftstoffe maskieren dabei zumeist den Geruch der anderen Inhaltstoffe, so dass beim Verbraucher ein angenehmer Geruchseindruck entsteht.

Insbesondere im Bereich Waschmittel sind Duftstoffe wichtige Bestandteile der Zusammensetzung, da die Wäsche sowohl im feuchten als auch im trockenen Zustand einen angenehmen und möglichst auch frischen Duft aufweisen soll. Man steht beim Einsatz von Duftstoffen grundsätzlich vor dem Problem, dass es sich bei diesen um mehr oder minder leicht flüchtige Verbindungen handelt, aber dennoch ein lange anhaltender Dufteffekt angestrebt wird. Insbesondere bei denjenigen Riechstoffen, die die frischen und leichten Noten des Parfüms darstellen und infolge ihres hohen Dampfdruck besonders schnell abdampfen, ist die gewünschte Langlebigkeit des Dufteindrucks kaum erreichbar.

Eine verzögerte Duftfreisetzung kann z.B. durch Träger-gebundenen Einsatz von Duftstoffen erfolgen. Eine Träger-gebundene Vorform eines Duftstoffes wird auch als "Pro-Fragrance" oder Duftspeicherstoff bezeichnet. In diesem Zusammenhang offenbart das US-Patent 6,949,680 die Verwendung bestimmter Phenyl- oder Pyridylketone als photoaktivierbare Substanzen, die in Gegenwart von Licht in einer photochemischen Fragmentierung ein terminales Alken als Aktivstoff freisetzen. Der genannte Aktivstoff besitzt beispielsweise eine duftgebende oder antimikrobielle Aktivität, die durch den photochemisch induzierten Zerfall erst verzögert und über einen längeren Zeitraum hinweg auf einer bestimmten Oberfläche freigesetzt wird.

WO 2009/118219 A1 offenbart photoaktivierbare Substanzen, welche eine Freisetzung von cyclischen Terpenen oder cyclischen Terpenoiden ermöglichen.

WO 2011/101180 offenbart die Verwendung bestimmter Ketone als photoaktivierbare Substanzen, die in Gegenwart von Licht in einer photochemischen Fragmentierung einen Aktivstoff freisetzen. Der genannte Aktivstoff besitzt beispielsweise eine duftgebende Aktivität, die durch den photochemisch induzierten Zerfall erst verzögert und über einen längeren Zeitraum hinweg auf einer bestimmten Oberfläche freigesetzt wird.

Aufgabe der vorliegenden Erfindung war die Bereitstellung photoaktivierbarer Substanzen als Duftspeicherstoffe, welche die verzögerte Freisetzung von Riechstoffketonen, insbesondere von Damascone erlauben.

Gelöst wurde diese Aufgabe durch eine Verbindung entsprechend der Formel (I) 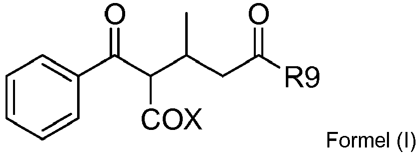 dadurch gekennzeichnet, dass
R9 für einen Kohlenwasserstoffrest mit wenigstens 5 C-Atomen steht;
X für H, -R11, -OR11, -NR11R12, -SR11 oder Halogen, vorzugsweise -OR11 steht, wobei
R11 und R12 unabhängig voneinander für Wasserstoff, eine lineare oder verzweigte, unsubstituierte Alkyl- oder Alkenylgruppe mit bis zu 20 C-Atomen, eine Arylgruppe mit 6 bis 14 C-Atomen, Alkylaryl- oder Arylalkylgruppe, wobei Alkyl und Aryl wie vorstehend definiert sind, stehen oder für eine (CH(R13)CH(R14)O)ₚ-R15 Gruppe stehen mit p=1-20 und wobei R13, R14 und R15 unabhängig voneinander für Wasserstoff, eine lineare oder verzweigte, unsubstituierte Alkylgruppe stehen.

Als Gruppe -COX ist im Sinne der vorliegenden Anmeldung eine Carbonylverbindung der allgemeinen Formel -(C=O)-X zu verstehen, wobei der Rest -X wie oben beschrieben definiert ist.

Es konnte überraschend gefunden werden, dass die erfindungsgemäßen Ketone besonders wirksame Duftspeicherstoffe sind, welche die verzögerte Freisetzung von Riechstoffketonen, insbesondere von Damascone erlauben. Die Anwendung der erfindungsgemäßen Ketone in Wasch-, Reinigungs- oder Pflegemitteln führte bei deren Anwendung zu einer verbesserten Langzeitduftwirkung, insbesondere im Zusammenhang mit der Textilbehandlung. Zum Beispiel konnte bei der Anwendung erfindungsgemäßer Ketone in einem Wäschebehandlungsmittels, wie z.B. Waschmittel sowie Weichspüler, eine verbesserte Langzeitduftwirkung der behandelten Wäsche gefunden werden. Ferner weisen entsprechende Produkte eine besonders gute Lagerstabilität auf. Die erfindungsgemäßen Mittel ermöglichen es zudem, die Gesamtmenge an Parfüm, welches im Mittel enthalten ist, zu reduzieren, und dennoch Geruchsvorteile auf den gewaschenen Textilien zu erzielen, insbesondere mit Blick auf das Frischeempfinden.

Das erfindungsgemäße Keton gemäß der Formel (I) ist bevorzugt für alle Damascone sowie Damascenone als Duftspeicherstoff geeignet. Durch Einwirkung von Licht umfassend die Wellenlängen von 200 bis 600 nm können die gespeicherten Ketone freigesetzt werden.

Gemäß einer bevorzugten Ausführungsform der Erfindung ist die Verbindung der Formel (I) dadurch gekennzeichnet, dass
R9 gleich 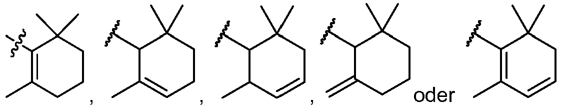 ist.

In einer weiteren bevorzugten Ausführungsform ist die Verbindung der Formel (I) dadurch gekennzeichnet, dass
R11 und R12 unabhängig voneinander für eine lineare oder verzweigte, unsubstituierte Alkylgruppe mit 1 bis 16 C-Atomen stehen oder für eine (CH(R13)CH(R14)O)ₚ-R15 Gruppe stehen mit p=1-10 und wobei R13, R14 und R15 unabhängig voneinander für Wasserstoff, Methyl oder Ethyl stehen.

Gemäß einer weiteren Ausführungsform der Erfindung sind Ketone entsprechend den nachfolgenden Formeln (II) bis (VIII) besonders bevorzugt

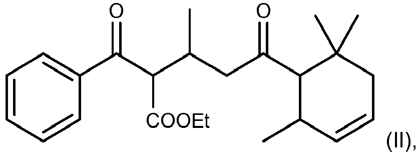

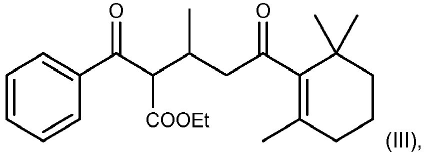

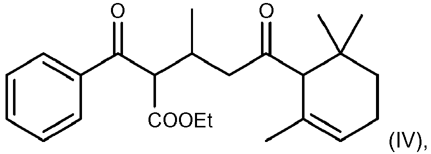

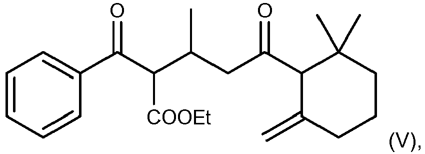

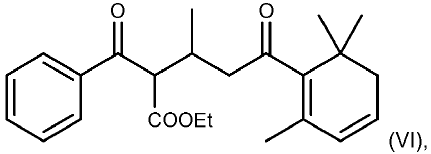

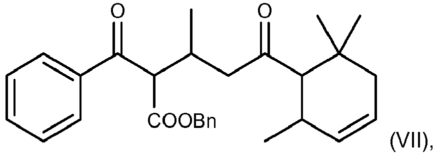

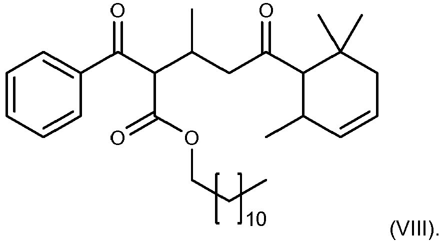

Die erfindungsgemäßen Ketone lassen sich stabil in die üblichen Wasch- oder Reinigungsmittelmatrices, in Kosmetika und bestehende Riechstoffkompositionen einarbeiten. Sie ermöglichen eine verzögerte Freisetzung der gespeicherten Duftstoffe, nämlich von Damascone in der alpha-, beta-, gamma- oder delta-Form sowie von Damascenone, insbesondere delta-Damascenone. Diese Ketone verleihen üblichen Wasch- oder Reinigungsmitteln sowie Kosmetika einen besonders lange anhaltenden Frischeeindruck. Insbesondere das getrocknete, gewaschene Textil profitiert von der guten Langzeitfrischeduftwirkung. Die langsame Freisetzung des gespeicherten Riechstoffes erfolgt nach Einwirkung von Licht (elektromagnetische Strahlung) umfassend die Wellenlängen von 200 bis 600 nm, wie in der nachfolgender Reaktionsgleichung vereinfacht veranschaulicht:

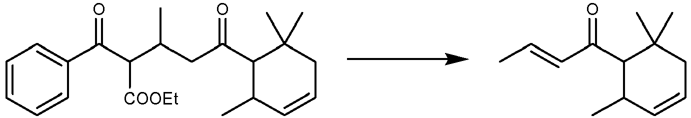

Ein weiterer Gegenstand der vorliegenden Erfindung ist ein Wasch- oder Reinigungsmittel, vorzugsweise ein Waschmittel, Weichspüler oder Waschhilfsmittel, enthaltend mindestens eine Verbindung gemäß der Erfindung.

Das genannte Keton ist in einem Aspekt vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten.

Geeignete Reinigungsmittel sind z.B. Reinigungsmittel für harte Oberflächen, wie vorzugsweise Geschirrspülmittel. Ebenso kann es sich um Reinigungsmittel wie z.B. Haushaltsreiniger, Allzweckreiniger, Fensterreiniger, Fußbodenreiniger usw. handeln. Vorzugsweise kann es sich um ein Produkt zur Reinigung von WC-Becken und Urinalen handeln, vorteilhafterweise um einen Spülreiniger zum Einhängen in das WC-Becken.

Gemäß einer bevorzugten Ausführungsform der Erfindung enthält das erfindungsgemäße Wasch- oder Reinigungsmittel mindestens ein Tensid, ausgewählt aus anionischen, kationischen, nichtionischen, zwitterionischen, amphoteren Tensiden oder Mischungen daraus.

Gemäß einer weiteren bevorzugten Ausführungsform der Erfindung liegt das erfindungsgemäße Mittel in fester oder flüssiger Form vor.

Ein weiterer Gegenstand der Erfindung ist ein kosmetisches Mittel, enthaltend mindestens eine Verbindung gemäß der Formel (I).

In einem Aspekt ist das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten.

Ein weiterer Gegenstand der Erfindung ist ein Luftpflegemittel (z.B. Raumlufterfrischer, Raumdeodorant, Raumspray usw.), enthaltend mindestens eine Verbindung gemäß der Formel (I).

In einem Aspekt ist das genannte Keton vorzugsweise in Mengen zwischen 0,0001 und 50 Gew.-%, vorteilhafterweise zwischen 0,001 und 5 Gew.-%, weiter vorteilhaft zwischen 0,01 und 3 Gew.-%, insbesondere zwischen 0,1 und 2 Gew.-%, jeweils bezogen auf das gesamte Mittel, enthalten.

Nach einer weiteren bevorzugten Ausführungsform der Erfindung sind in einem erfindungsgemäßen Mittel (d.h. Wasch- oder Reinigungsmittel, kosmetisches Mittel, oder Luftpflegemittel) zusätzliche Duftstoffe enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Haftfeste Riechstoffe, die im Rahmen der vorliegenden Erfindung mit Vorteil einsetzbar sind, sind beispielsweise ätherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch höhersiedende bzw. feste Riechstoffe natürlichen oder synthetischen Ursprungs können im Rahmen der vorliegenden Erfindung als haftfeste Riechstoffe bzw. Riechstoffgemische, also Duftstoffe eingesetzt werden. Zu diesen Verbindungen zählen die nachfolgend genannten Verbindungen sowie Mischungen aus diesen: Ambrettolid, alpha-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, alpha-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-beta-naphthylketon, Methyl-n-nonylacetaldehyd, Methyl-n-nonylketon, Muskon, beta-Naphtholethylether, beta-Naphtholmethylether, Nerol, Nitrobenzol, n-Nonylaldehyd, Nonylakohol, n-Octylaldehyd, p-Oxy-Acetophenon, Pentadekanolid, beta-Phenylethylalkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, gamma-Undecalacton, Vanillin, Veratrumaldehyd, Zimtaldehyd, Zimatalkohol, Zimtsäure, Zimtsäureethylester, Zimtsäurebenzylester. Zu den leichter flüchtigen Duftstoffen zählen insbesondere die niedriger siedenden Riechstoffe natürlichen oder synthetischen Ursprung, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -Propionat, Menthol, Menthon, Methyl-n-heptenon, Phellandren, Phenylacetaldehyd, Terpinylacetat, Zitral, Zitronellal.

Nach einer weiteren bevorzugten Ausführungsform weist das erfindungsgemäße Mittel (d.h. Wasch- oder Reinigungsmittel, kosmetisches Mittel, oder Luftpflegemittel), wenigstens eine, vorzugsweise mehrere, aktive Komponenten, insbesondere wasch-, pflege-, reinigungsaktive und/oder kosmetische Komponenten auf, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Einlaufverhinderer, Elektrolyte, Enzyme, Farbschutzstoffe, Färbemittel, Farbstoffe, Farbübertragungsinhibitoren, Fluoreszenzmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, soil-release-Wirkstoffe, UV-Schutz-Substanzen, Viskositätsregulatoren, Verdickungsmittel, Verfärbungsinhibitoren, Vergrauungsinhibitoren, Vitamine und/oder Weichspüler. Im Sinne dieser Erfindung beziehen sich Angaben für das erfindungsgemäße Mittel in Gew.-%, wenn nicht anders angegeben, auf das Gesamtgewicht des erfindungsgemäßen Mittels.

Die Mengen der einzelnen Inhaltsstoffe in den erfindungsgemäßen Mitteln (d.h. Wasch- oder Reinigungsmittel, kosmetisches Mittel oder Luftpflegemittel) orientieren sich jeweils am Einsatzzweck der betreffenden Mittel und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der erfindungsgemäßen Mittel wird man beispielsweise den Tensidgehalt höher oder niedriger wählen. Üblicherweise kann z.B. der Tensidgehalt beispielsweise von Waschmitteln zwischen 10 und 50 Gew.-%, vorzugsweise zwischen 12,5 und 30 Gew.-% und insbesondere zwischen 15 und 25 Gew.-% betragen, während z.B. Reinigungsmittel für das maschinelle Geschirrspülen z.B. zwischen 0,1 und 10 Gew.-%, vorzugsweise zwischen 0,5 und 7,5 Gew.-% und insbesondere zwischen 1 und 5 Gew.-% Tenside enthalten können.

Die erfindungsgemäßen Mittel (d.h. Wasch- oder Reinigungsmittel, kosmetisches Mittel oder Luftpflegemittel) können Tenside enthalten, wobei bevorzugt anionische Tenside, nichtionische Tenside und deren Gemische, aber auch kationische Tenside in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen, sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺X⁻ ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X⁻ für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden z.B. mit Dimethylsulfat quaterniert. In Frage kommende QAV sind beispielweise Benzalkoniumchlorid (N Alkyl-N,N dimethyl-benzylammoniumchlorid), Benzalkon B (m,p-Dichlorbenzyldimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzyl-ammoniumchlorid), Dialkyldimethylammoniumchloride wie Di-n-decyl-dimethyl-ammoniumchlorid, Didecyldimethylammoniumbromid, Dioctyl-dimethyl-ammoniumchlorid, 1-Cetylpyridiniumchlorid und Thiazolinjodid sowie deren Mischungen. Bevorzugte QAV sind die Benzalkoniumchloride mit C₈-C₂₂-Alkylresten, insbesondere C₁₂-C₁₄-Alkylbenzyl-dimethylammoniumchlorid.

Bevorzugte Esterquats sind Methyl-N-(2-hydroxyethyl)-N,N-di(talgacyl-oxyethyl)ammonium-metho-sulfat, Bis-(palmitoyl)-ethyl-hydroxyethyl-methyl-ammonium-methosulfat oder Methyl-N,N-bis(acyl-oxyethyl)-N-(2-hydroxyethyl)ammonium-methosulfat. Handelsübliche Beispiele sind die von der Firma Stepan unter dem Warenzeichen Stepantex vertriebenen Methylhydroxyalkyldialkoyloxyalkylammoniummethosulfate oder die unter dem Handelsnamen Dehyquart bekannten Produkte der Firma BASF SE beziehungsweise die unter der Bezeichnung Rewoquat bekannten Produkte des Herstellers Evonik.

Tenside sind in den erfindungsgemäßen Mitteln (d.h. Wasch- oder Reinigungsmittel, kosmetisches Mittel, oder Luftpflegemittel) in Mengenanteilen von vorzugsweise 5 Gew.-% bis 50 Gew.-%, insbesondere von 8 Gew.-% bis 30 Gew.-%, enthalten. Insbesondere in Wäsche-Nachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein erfindungsgemäßes Mittel, insbesondere Wasch- oder Reinigungsmittel, enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Organische Buildersubstanzen können, falls gewünscht, in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Erfindungsgemäße Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, falls gewünscht, in Mengen von bis zu 50 Gew.-%, vorzugsweise nicht über 40 Gew.-% und in flüssigen Mitteln insbesondere von 1 Gew.-% bis 5 Gew.-%, eingesetzt werden. Unter diesen sind die kristallinen Natriumalumosilikate in Waschmittelqualität, insbesondere Zeolithe A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁·yH₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl beta- als auch delta-Natriumdisilikate (Na₂Si₂O₅·yH₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind, falls gewünscht, in den erfindungsgemäßen Mitteln vorzugsweise in Mengen bis zu 60 Gew.-%, insbesondere von 5 Gew.-% bis 40 Gew.-%, enthalten. Erfindungsgemäße Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wässriger Lösungen, die 3 Gew.-% bis 10 Gew.-% Wasserstoffperoxid enthalten, ggf. eingesetzt. Falls ein erfindungsgemäßes Mittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten bzw. Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxo-hexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten sein.

Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind, falls gewünscht, in den erfindungsgemäßen Mitteln vorzugsweise in Mengen nicht über 5 Gew.-%, insbesondere von 0,2 Gew.-% bis 2 Gew.-%, enthalten.

Die Mittel können ggf. als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen.

Zu den geeigneten Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäure-alkylendiamiden. Mit Vorteilen werden auch Gemische aus verschiedenen Schauminhibitoren verwendet, zum Beispiel solche aus Silikonen, Paraffinen oder Wachsen. Vorzugsweise sind die Schauminhibitoren, insbesondere Silikon- und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem Mittel, das diese öl- und fettlösende Komponente enthält, gewaschen wurde. Zu den bevorzugten öl- und fettlösenden Komponenten zählen beispielsweise nicht-ionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Mittel können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt werden.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Apfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind optional in den erfindungsgemäßen Mitteln vorzugsweise nicht über 20 Gew.-%, insbesondere von 1,2 Gew.-% bis 17 Gew.-%, enthalten.

Die Herstellung fester erfindungsgemäßer Mittel (d.h. insbesondere Wasch- oder Reinigungsmittel) bereitet keine Schwierigkeiten und kann im Prinzip in bekannter Weise, zum Beispiel durch Sprühtrocknen oder Granulation, erfolgen, wobei optionale Persauerstoffverbindung und optionaler Bleichkatalysator gegebenenfalls später zugesetzt werden. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Mittel bereitet ebenfalls keine Schwierigkeiten und kann ebenfalls in bekannter Weise erfolgen.

Die Herstellung der erfindungsgemäßen Ketone wird im Beispielteil exemplarisch anhand der Herstellung eines delta-Damascone-enthaltenden Duftspeicherstoffes beschrieben. Über diese prinzipielle Syntheseroute sind auch die anderen Verbindungen entsprechend der Formel (I) darstellbar.

Gemäß einer bevorzugten Ausführungsform kann die erfindungsgemäße Lehre dazu eingesetzt werden, den Parfümanteil in Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder gar nicht verwenden können.

Ein bevorzugtes erfindungsgemäßes festes, insbesondere pulverförmiges Waschmittel kann neben dem erfindungsgemäßen Keton insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-30 Gew.-%
- Nichtionische Tenside, wie vorzugsweise Fettalkoholpolyglycolether, Alkylpolyglucosid, Fettsäureglucamid z.B. in Mengen von vorzugsweise 0,5-15 Gew.-%
- Gerüststoffe, wie z.B. Zeolith, Polycarboxylat, Natriumcitrat, in Mengen von z.B. 0-70 Gew.-%, vorteilhafterweise 5-60 Gew.-%, vorzugsweise 10-55 Gew.-%, insbesondere 15-40 Gew.-%,
- Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
- Bleichmittel, wie z.B. Natriumperborat, Natriumpercarbonat, in Mengen von z.B. 0-30 Gew.-% vorteilhafterweise 5-25 Gew.-%, vorzugsweise 10-20 Gew.-%,
- Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
- Stabilisatoren, z.B. Phosphonate, vorteilhafterweise 0-1 Gew.-%,
- Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
- Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
- Vergrauungsinhibitor, z.B. Carboxymethylcellulose, vorteilhafterweise 0-1 Gew.-%,
- Verfärbungsinhibitor, z.B. Polyvinylpyrrolidon-Derivate, vorzugsweise 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0-0,4 Gew.-%, insbesondere 0,1-0,3 Gew.-%,
- ggf. weitere Duftstoffe
- ggf. Wasser
- ggf. Seife
- ggf. Bleichaktivatoren
- ggf. Cellulosederivate
   ggf. Schmutzabweiser,
Gew.-% jeweils bezogen auf das gesamte Mittel.

In einer anderen bevorzugten Ausführungsform der Erfindung liegt das Mittel in flüssiger Form vor, vorzugsweise in Gelform. Bevorzugte flüssige Wasch- oder Reinigungsmittel sowie Kosmetika haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein bevorzugtes erfindungsgemäßes flüssiges, insbesondere gelförmiges Waschmittel kann neben dem erfindungsgemäßen Keton insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Duftstoffe
- ggf. Stabilisatoren,
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein bevorzugter erfindungsgemäßer flüssiger Weichspüler kann neben dem erfindungsgemäßen Keton insbesondere noch Komponenten enthalten, die ausgewählt sind aus den folgenden:
- Kationische Tenside, wie insbesondere Esterquats, z.B. in Mengen von 5-30 Gew.-%,
- Cotenside, wie z.B. Glycerolmonostearat, Stearinsäure, Fettalkohole, Fettalkoholethoxylate, z.B. in Mengen von 0-5 Gew.-%, vorzugsweise 0,1-4 Gew.-%,
- Emulgatoren, wie z.B. Fettaminethoxylate, z.B. in Mengen von 0-4 Gew.-%, vorzugsweise 0,1-3 Gew.-%,
- ggf. weitere Duftstoffe
- Farbstoffe, vorzugsweise im ppm-Bereich
- Stabilisatoren, vorzugsweise im ppm-Bereich
- Lösemittel, wie z.B. Wasser, in Mengen von vorzugsweise 60-90 Gew.-%,
- Gew.-% jeweils bezogen auf das gesamte Mittel.

Ein weiterer Gegenstand der Erfindung ist ein kosmetisches Mittel, wobei das kosmetische Mittel eine erfindungsgemäße Verbindung der Formel (I) enthält.

Ein weiterer Gegenstand der Erfindung ist ein Verfahren zur lang anhaltenden Beduftung von Oberflächen, wobei eine erfindungsgemäße Verbindung der Formel (I) oder ein erfindungsgemäßes Wasch- oder Reinigungsmittel auf die zu beduftende Oberfläche (z.B. Textil, Geschirr, Fußboden) aufgebracht wird und die genannte Oberfläche anschließend einer elektromagnetischen Strahlung umfassend die Wellenlängen von 200 bis 600 nm ausgesetzt wird.

Ein weiterer Gegenstand der Erfindung ist ein Verfahren zur lang anhaltenden Raumbeduftung, wobei ein erfindungsgemäßes Luftpflegemittel einer elektromagnetischen Strahlung umfassend die Wellenlängen von 200 bis 600 nm ausgesetzt wird.

### Beispiel 1: Synthese des Benzoylessigsäureethylesters

Darstellung eines Ketons der Formel (I):

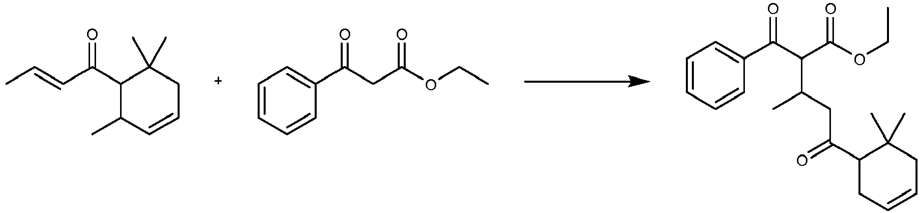

Jeweils 17,3 g (90 mmol) Benzoylessigsäureethylester (CAS Nr.: 94-92-0) und delta-Damascone (90 mmol, CAS Nr.: 57378-68-4) wurden in 15 ml Chloroform unter Stickstoffatmosphäre gelöst. Anschließend wurden 0,24 g Eisen(III)chlorid•6H₂O (0,9 mmol) hinzugefügt, wodurch sich die Lösung sofort braun färbte.

Es wurde 24 h gerührt, anschließend wurde die Lösung filtriert. Das rotbraune Rohprodukt wird in Chloroform gelöst und über eine mit neutralem Aluminumoxid gefüllte Säule erneut filtriert.

Erhalten wurden 28,2 g orangefarbenes, klares, viskoses Produkt.

¹H-NMR (CDCl₃): m 8,0 (2H); m 7,6 (1H); m 7,5 (2H); m 5,5 (1H); dbr 5,4 (1H); m 4,6 (1H); m 4,1 (2H); 2,9 (2H); m 2,6-2,5 (2H); m 2,2 (1H); m 1,9 (1H); m 1,7 (1H); m 1,1-0,9 (12H), dd (3H).

IR: 3500, 3000, 1739, 1705, 1687, 1598, 1580.

Das auf diese Weise hergestellte Keton zeigte bei der Anwendung in Waschmitteln und Weichspülern bei der Textilbehandlung eine sehr gute Duftwirkung. Insbesondere wurde eine bessere Dauerhaftigkeit des Dufteindruckes auf der damit gewaschenen und danach getrockneten Wäsche gefunden, verglichen mit Waschmitteln und Weichspülern die eine äquivalente Menge delta-Damascone enthielten, ansonsten aber gleichgestaltet waren. Der frische Dufteindruck der Textilien hielt deutlich länger vor, sowohl nach Leinentrocknung wie insbesondere nach Trocknung im Maschinentrockner.

### Beispiel 2: Benzyl-2-benzoyl-3-methyl-5-oxo-5-(2,6,6-trimethylcyclohex-3-enyl)-pentanoat

### 1. Stufe Benzoylessigsäurebenzylester

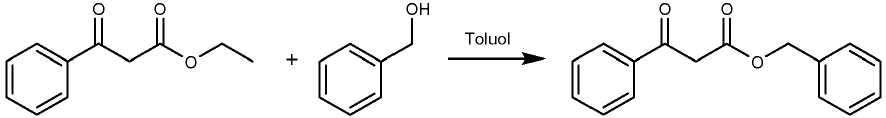

In einem 100 ml Rundkolben wurde 7,45 mmol Benzoylessigsäureethylester und 8,90 mmol Benzylalkohol in 50 ml Toluol gelöst und 24 h zum Rückfluss erhitzt. Das Reaktionsgemisch wurde auf Raumtemperatur abgekühlt und das Lösungsmittel unter vermindertem Druck entfernt. Das Produkt wurde ohne weitere Reinigung in die nächste Stufe eingesetzt.

### 2. Stufe:

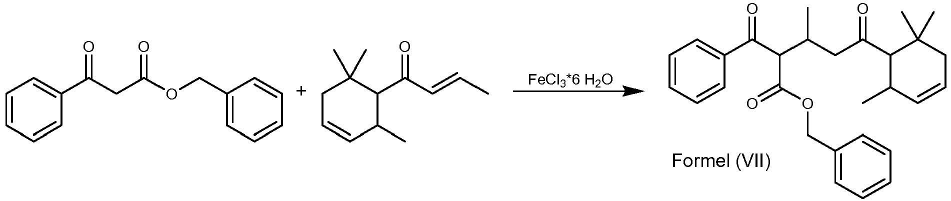

In einem 25 ml Rundkolben wurde ein Gemisch aus 12 mmol Benzoylessigsäurebenzylester, 12 mmol δ-Damascon und 2.4 mmol Eisen(III)-chlorid Hexahydrat für 24 h bei 50 °C gerührt. Anschließend wurde das Rohprodukt säulenchromatographisch aufgereinigt (Methyl-tert.butylether : Cyclohexan, 1:4 R_{f} = 0,41). Die Ausbeute, bezogen auf das eingesetzte Rohprodukt betrug 80 %.

### Beispiel 3: Dodecyl-2-benzoyl-3-methyl-5-oxo-5-(2,6,6-trimethylcyclohex-3-enyl)-pentanoat

### 1. Stufe Benzoylessigsäuredodecylester

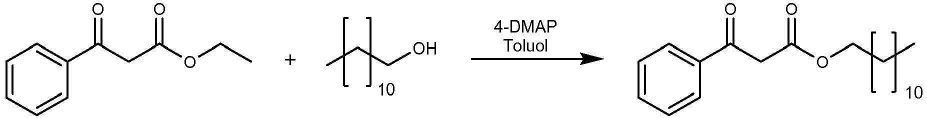

In einem 100 ml Rundkolben wurden 10 mmol Benzoylessigsäureethylester, 11,9 mmol 1-Dodecanol und 1 mmol 4-Dimethylaminoryridin in 50 ml Toluol vorgelegt. Das entstehende Ethanol wurde mit dem Lösungsmittel abdestilliert. Das entstandene Produkt wurde säulenchromatographisch aufgereinigt (Acetessigester : Cyclohexan: 1:10, R_{f} = 0,52), wobei die Ausbeute 76 % betrug.

### 2. Stufe

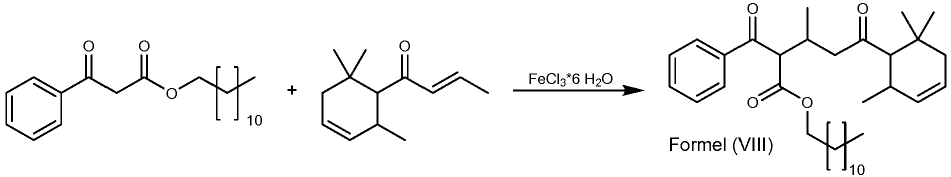

In einem 25 ml Rundkolben wurde ein Gemisch aus 10 mmol Benzoylessigsäurebenzylester, 10 mmol δ-Damascon und 1 mmol Eisen(III)-Chlorid Hexahydrat für 24 h bei 50 °C gerührt. Anschließend wurde das Rohprodukt säulenchromatographisch aufgereinigt (Methyl-tert.butylether : Cyclohexan, 1:4 R_{f} = 0,43). Die Ausbeute betrug 72 %.

### Beispiel 4: Freisetzungsverhalten

Die Testsubstanzen wurden mol-gleich in Bezug auf den darin enthaltenen Riechstoff in einen Weichspüler einformuliert, dieser wurde im Spülgang eines Waschprozesses verwendet. Die so behandelte Wäsche wurde nach dem Trocknen für 1 bis 7 h dem Sonnenlicht ausgesetzt und anschließend von zehn geruchlich geschulten Personen abgerochen, wobei jede Probe in zwei voneinander unabhängigen Durchgängen bewertet wurde. Die Duftintensität wird durch eine Skala von 1 bis 10 (10: sehr stark, 1: sehr schwach) wiedergegeben.

Untersucht wurden der reine Riechstoff delta-Damascone (Referenz) und die aus dem Stand der Technik bekannte Verbindung mit der Formel (IX) 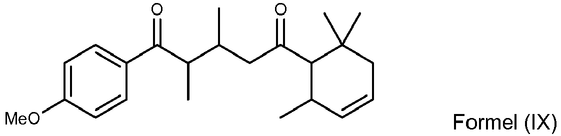 und die erfindungsgemäßen Ketone der Formeln (II) und (VIII):

| Probe | Dosierung [Gew.-%] | Duftintensität nach 1 h Sonne | Duftintensität nach 3 h Sonne | Duftintensität nach 5 h Sonne | Duftintensität nach 7 h Sonne |
|---|---|---|---|---|---|
| Referenz | 0,4 | 3 | 2 | 1 | 2 |
| Keton IX | 0,74 | 3 | 3 | 3 | 3 |
| Keton II | 0,80 | 6 | 6 | 6 | 7 |
| Keton VIII | 1,65 | 7 | 6 | 7 | 7 |

Es zeigt sich, dass die erfindungsgemäße Verbindung den bekannten Substanzen im Hinblick auf die Duftintensität überlegen ist.

## Patentansprüche

1. Verbindung entsprechend der Formel (I) 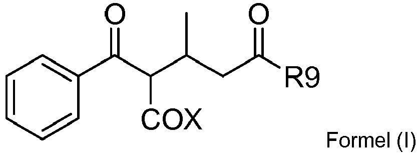 **dadurch gekennzeichnet, dass**
R9 für einen Kohlenwasserstoffrest mit wenigstens 5 C-Atomen steht;
X für H, -R11, -OR11, -NR11R12, -SR11 oder Halogen steht, wobei
R11 und R12 unabhängig voneinander für Wasserstoff, eine lineare oder verzweigte, unsubstituierte Alkyl- oder Alkenylgruppe mit bis zu 20 C-Atomen, eine Arylgruppe mit 6 bis 14 C-Atomen, Alkylaryl- oder Arylalkylgruppe, wobei Alkyl und Aryl wie vorstehend definiert sind, stehen oder für eine (CH(R13)CH(R14)O)ₚ-R15 Gruppe stehen mit p=1-20 und wobei R13, R14 und R15 unabhängig voneinander für Wasserstoff, eine lineare oder verzweigte, unsubstituierte Alkylgruppe stehen.

2. Verbindung nach Anspruch 1, **dadurch gekennzeichnet, dass**
R9 gleich 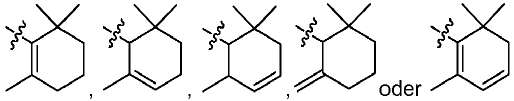 ist.

3. Verbindung nach Anspruch 1 oder 2, **dadurch gekennzeichnet, dass**
R11 und R12 unabhängig voneinander für eine lineare oder verzweigte, unsubstituierte Alkylgruppe mit 1 bis 16 C-Atomen stehen oder für eine (CH(R13)CH(R14)O)ₚ-R15 Gruppe stehen mit p=1-10 und wobei R13, R14 und R15 unabhängig voneinander für Wasserstoff, Methyl oder Ethyl stehen.

4. Verbindung nach einem der Ansprüche 1 bis 3, entsprechend einer der folgenden Formeln (II) bis (VIII):

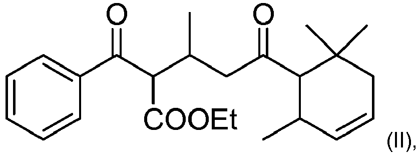

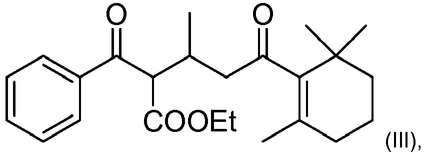

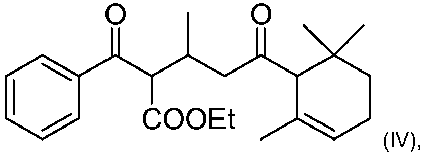

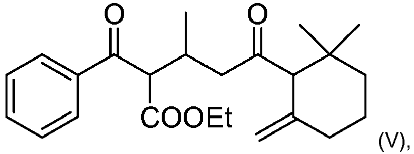

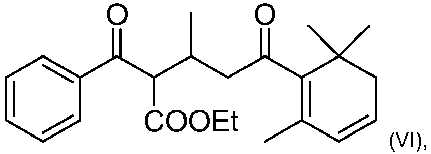

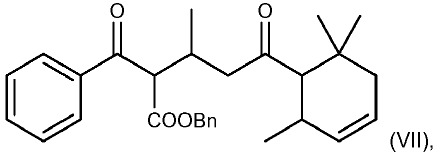

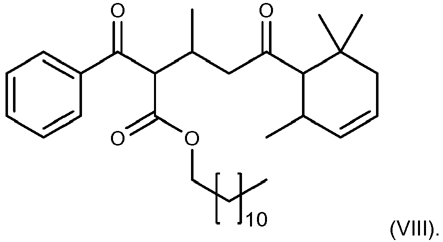

5. Wasch- oder Reinigungsmittel, enthaltend mindestens eine Verbindung nach einem der Ansprüche 1 bis 4.

6. Wasch- oder Reinigungsmittel nach Anspruch 5, **dadurch gekennzeichnet, dass**
(1) es mindestens ein Tensid ausgewählt aus der Gruppe bestehend aus anionischen, kationischen, nichtionischen, zwitterionischen, amphoteren Tensiden oder Mischungen daraus enthält; und/oder
(2) es in fester oder flüssiger Form vorliegt.

7. Luftpflegemittel, enthaltend mindestens eine Verbindung nach einem der Ansprüche 1 bis 4.

8. Kosmetisches Mittel, enthaltend mindestens eine Verbindung nach einem der Ansprüche 1 bis 4.

9. Verfahren zur langanhaltenden Beduftung von Oberflächen, **dadurch gekennzeichnet, dass** eine Verbindung nach einem der Ansprüche 1 bis 4 oder ein Mittel nach Anspruch 5 oder 6 auf die zu beduftende Oberfläche aufgebracht wird und die genannte Oberfläche anschließend einer elektromagnetischen Strahlung umfassend die Wellenlängen von 200 bis 600 nm ausgesetzt wird.

10. Verfahren zur langanhaltenden Raumbeduftung, **dadurch gekennzeichnet, dass** ein Mittel nach Anspruch 7 enthalten ist, das einer elektromagnetischen Strahlung umfassend die Wellenlängen von 200 bis 600 nm ausgesetzt wird.

## Claims

1. A compound corresponding to formula (I) 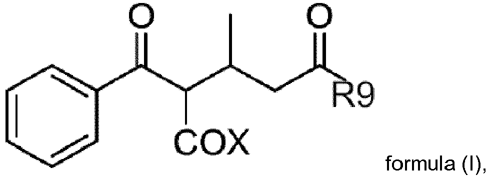 **characterized in that**
R9 represents a hydrocarbon group having at least 5 C atoms;
X represents H, -R11, -OR11, -NR11R12, -SR11 or halogen, wherein
R11 and R12 represent, independently of one another, hydrogen, a linear or branched, unsubstituted alkyl or alkenyl group having up to 20 C atoms, an aryl group having 6 to 14 C atoms, an alkylaryl or arylalkyl group, whereby alkyl and aryl being as defined above, or represent a (CH(R13)CH(R14)O)ₚ-R15 group, where p=1-20, and wherein R13, R14 and R15 represent, independently of one another, hydrogen, a linear or branched, unsubstituted alkyl group.

2. The compound according to claim 1, **characterized in that** R9 is

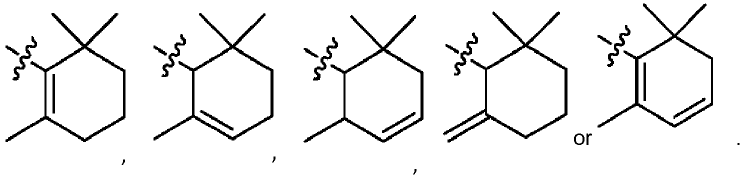

3. The compound according to claim 1 or 2, **characterized in that** R11 and R12 represent, independently of one another, a linear or branched, unsubstituted alkyl group having 1 to 16 C atoms, or a (CH(R13)CH(R14)O)ₚ-R15 group, where p=1-10, and wherein R13, R14 and R15 represent, independently of one another, hydrogen, methyl or ethyl.

4. The compound according to one of claims 1 to 3, corresponding to one of the following formulae (II) to (VIII):

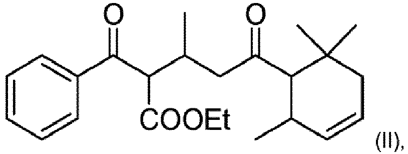

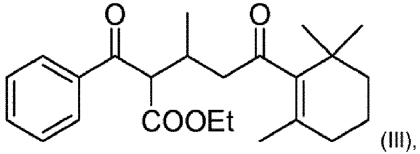

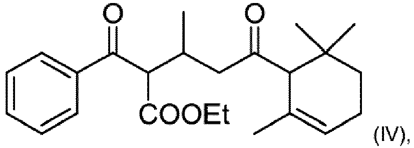

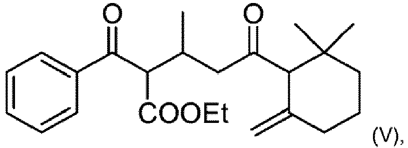

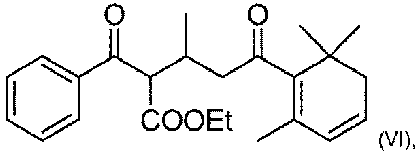

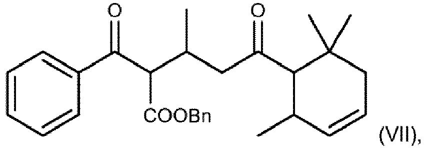

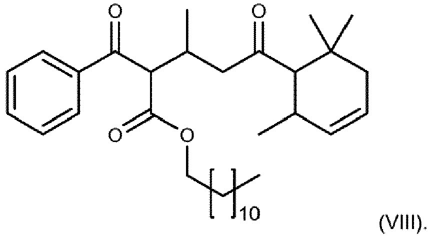

5. A washing or cleaning agent containing at least one compound according to one of claims 1 to 4.

6. The washing or cleaning agent according to claim 5, **characterized in that**
(1) it contains at least one surfactant selected from the group consisting of anionic, cationic, non-ionic, zwitterionic, amphoteric surfactants or mixtures thereof; and/or
(2) it is in solid or liquid form.

7. An air-freshening agent containing at least one compound according to one of claims 1 to 4.

8. A cosmetic agent containing at least one compound according to one of claims 1 to 4.

9. A method for long-lastingly fragrancing surfaces, **characterized in that** a compound according to one of claims 1 to 4 or an agent according to claim 5 or 6 is applied to the surface to be fragranced and said surface is then exposed to electromagnetic radiation having a wavelength of from 200 to 600 nm.

10. A method for long-lastingly fragrancing a room, **characterized in that** an agent according to claim 7 is contained, which agent is exposed to electromagnetic radiation having a wavelength of from 200 to 600 nm.

## Revendications

1. Composé selon la formule (I) 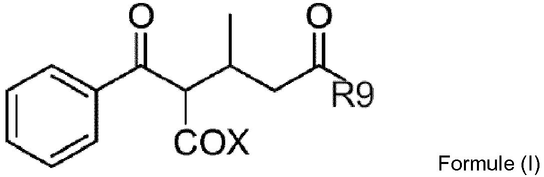 **caractérisé en ce que**
R9 représente un résidu hydrocarbure ayant au moins 5 atomes de carbone ;
X représente H, -R11, -OR11, -NR11R12, -SR11 ou un halogène, où R11 et R12 représentent, indépendamment l'un de l'autre, un atome d'hydrogène, un groupe alkyle ou alcényle linéaire ou ramifié non-substitué, ayant jusqu'à 20 atomes de carbone, un groupe aryle, ayant 6 à 14 atomes de carbone, un groupe alkylaryle ou arylalkyle, où alkyle et aryle sont définis comme précédemment, ou représentent un groupe (CH(R13)CH(R14)O)ₚ-R15 avec p = 1-20 et où R13, R14 et R15, indépendamment les uns des autres, un atome d'hydrogène ou un groupe alkyle linéaire ou ramifié non-substitué.

2. Composé selon la revendication 1, **caractérisé en ce que** R9 est

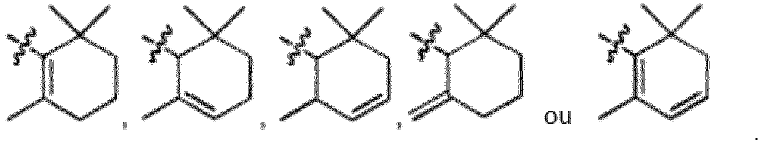

3. Composé selon la revendication 1 ou 2, **caractérisé en ce que** R11 et R12, indépendamment l'un de l'autre, représente un groupe alkyle linéaire ou ramifié non-substitué ayant 1 à 16 atomes de carbone ou un groupe (CH(R13)CH(R14)O)ₚ-R15 avec p = 1-10, et où R13, R14 et R15, indépendamment les uns des autres, représentent un atome d'hydrogène, un groupe méthyle ou éthyle.

4. Composé selon l'une des revendications 1 à 3, correspondant à une des formules suivantes (II) à (VIII) :

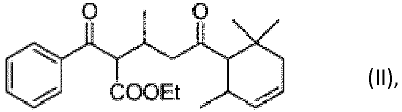

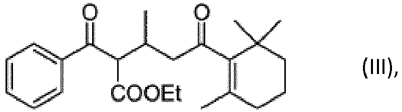

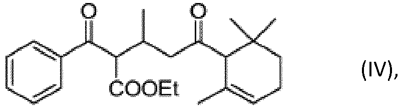

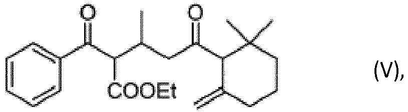

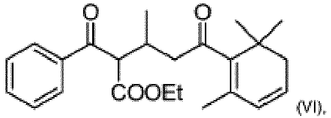

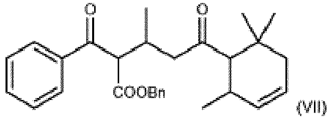

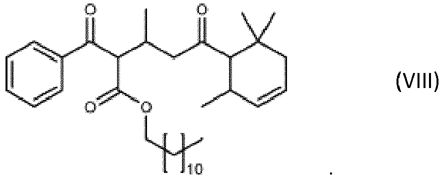

5. Produit de lavage ou de nettoyage contenant au moins un composé selon l'une des revendications 1 à 4.

6. Produit de lavage ou de nettoyage selon la revendication 5, **caractérisé en ce que**
(1) il contient au moins un tensioactif choisi dans le groupe constitué de tensioactifs anioniques, cationiques, non-ioniques, zwitterioniques, amphotères, ou de leurs mélanges ; et/ou
(2) il se présente sous forme solide ou liquide.

7. Produit de purification d'air contenant au moins un composé selon l'une des revendications 1 à 4.

8. Produit cosmétique contenant au moins un composé selon l'une des revendications 1 à 4.

9. Procédé pour parfumer durablement une surface, **caractérisé en ce qu'**un composé selon l'une des revendications 1 à 4 ou un produit selon la revendication 5 ou 6 est appliqué sur la surface à parfumer, puis ladite surface est exposée à un rayonnement électromagnétique comprenant les longueurs d'onde de 200 à 600 nm.

10. Procédé pour parfumer durablement une surface, **caractérisé en ce qu'**un produit selon la revendication 7 est contenu, qui est exposé à un rayonnement électromagnétique comprenant les longueurs d'onde de 200 à 600 nm.
